# EUROPEAN PATENT APPLICATION

(11) **EP 4 505 996 A1**
(43) Date of publication of application: **12.02.2025**
(21) Application number: 24184673.2
(22) Date of filing: 26.06.2024
(51) Int. Cl.: A61K 9/00, A61K 31/4015, A61K 47/12, A61K 47/38, A61P 25/08

(54) **AN ORAL SOLUTION COMPRISING BRIVARACETAM**

(30) Priority: 11.08.2023 TR 202309659
(71) Applicant: Sanovel Ilac Sanayi ve Ticaret A.S., Sisli - Istanbul (TR)
(72) Inventor: SUNEL, Fatih, Istanbul (TR); BILGEHAN ATAK, Fadime, Istanbul (TR); ATAMAN, Seval, Istanbul (TR); YAZICI, Ozlem, Istanbul (TR)
(74) Representative: Genç Ilhan, Oznur

(57) **Abstract**

The present invention relates to an oral solution comprising brivaracetam and at least one pharmaceutically acceptable excipient wherein at least one cellulose derivative excipient is present. Furthermore, the oral solution is also obtained by using an effective process which is simple, rapid, cost effective, time-saving and industrially convenient process.

## Description

### Field of the Invention

The present invention relates to an oral solution comprising brivaracetam and at least one pharmaceutically acceptable excipient wherein at least one cellulose derivative excipient is present. Furthermore, the oral solution is also obtained by using an effective process which is simple, rapid, cost effective, time-saving and industrially convenient process.

### Background of the Invention

The chemical name of brivaracetam is (2S)-2-[(4R)-2-oxo-4-propyl-pyrrolidin-1-yl]butanamide and its chemical structure is shown in the Formula I.

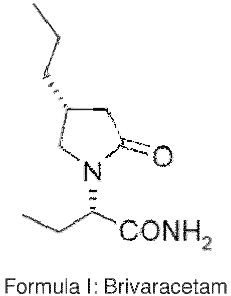

Brivaracetam is a white to off-white crystalline powder. It is very soluble in water, buffer (pH 1.2, 4.5, and 7.4), ethanol, methanol, and glacial acetic acid. It is freely soluble in acetonitrile and acetone and soluble in toluene. It is very slightly soluble in n-hexane.

Brivaracetam Oral Solution was launched in the United States on February 18, 2016. The original research is UCB INC, and the trade name is BRIVIACT.

We have found that Brivaracetam is extremely prone to increase the viscosity of the liquid due to its high adhesion during the preparation process for an oral solution. Also, during the process, problems such as fluidity and homogeneity appear. These problems lead to both solubility and stability problems.

Oral solution of brivaracetam is known in the prior art. There still remains a need in the art to provide an improved an oral solution comprising brivaracetam. In the present invention, the oral solution is created to overcome the above problems and provides additional advantages to the relevant field of art. Other advantages and embodiments of the present invention will be clarified in the following description.

### Detailed Description of the Invention

The main object of the present invention is to provide a brivaracetam oral solution has simple preparation technique, convenient administration, quick effect, good stability, which improves patient compliance.

Another object of the present invention is to solve the problem of oral liquid preparation technical, claims a preparation process of oral liquid safe taking and good taste.

In fact, active pharmaceutical agents in liquid forms are more susceptible to chemical and physical instability than in the solid state. In addition to the solubility, the active pharmaceutical agent needs to be physically and chemically stable in the oral solution. Trace amounts of impurities from active pharmaceutical agents or excipients and the pH of the solution may cause the degradation of the active pharmaceutical agent and this may cause an increase in instability when the solution is consistently introduced into the atmosphere.

Brivaracetam is unstable in aqueous solution and easily forms degradation products. It is necessary to use excipients with low toxicity at the lowest concentration. The use of small amounts of some excipients may be insufficient to reach the level of physicochemical stability. It is difficult to find excipients that are both non-toxic and provide effective stability even at low concentrations.

We have found that when at least one cellulose-derived excipient is present in the oral solution, degradation products are not formed in the brivaracetam solution and the solution can retain its color in the long term, provided it is stable.

According to one embodiment of this invention, an oral solution comprising brivaracetam and at least one pharmaceutically acceptable excipient wherein at least one cellulose derivative excipient is present.

With the combined use of cellulose derivatives and suitable tonicity adjusting agents ( for example : glycerin), we can mention that the viscosity can be optimized to ensure product stability. By providing optimum viscosity, the product's ability to be taken with a syringe is also meaningful in terms of patient compliance. In this way, its applicability to the patient is facilitated.

The viscosity agent is cellulose derivative excipient in the present invention. The viscosity agent is microcrystalline cellulose, sodium carboxymethyl cellulose, hydroxypropyl methyl cellulose, hydroxypropyl cellulose or mixtures thereof.

According to one embodiment of this invention, the viscosity agents are microcrystalline cellulose and sodium carboxymethyl cellulose.

According to one embodiment of this invention, the viscosity agent is hydroxypropyl methyl cellulose.

According to one embodiment of this invention, the viscosity agent is hydroxypropyl cellulose.

According to an embodiment of the present invention, the amount of viscosity agents is 0.1% to 2.0% by weight in the total composition. We found that even at low concentration it helps stability.

According to an embodiment of the present invention, the amount of brivaracetam is 0.1% to 7.0% by weight in the total composition.

According to an embodiment of the present invention, the amount of brivaracetam is 0.1% to 5.0% by weight in the total composition, preferably 0.2% to 3.0% by weight in the total composition, preferably 0.3% to 1.8% by weight in the total composition.

According to an embodiment of the present invention, Brivaracetam is 10mg/ml in the oral solution.

As used here in, 'particle size' means the cumulative volume size distribution as tested by any conventionally accepted method such as the laser diffraction method (i.e. Malvern analysis). The term d (0.1) means, the size at which 10% by volume of the particles are finer and d (0.5) means the size at which 50% by volume of the particles are finer and d (0.9) means the size at which %90 by volume of the particles is finer.

According to one embodiment of this invention, brivaracetam has a d (0.9) particle size between 100 µm to 300 µm.

According to one embodiment of this invention, brivaracetam has a d (0.9) particle size between 104 µm to 125 µm or between 126 µm to 138 µm or between 139 µm to 152 µm or between 153 µm to 167 µm or between 168 µm to 183 µm or between 184 µm to 198 µm or between 203 µm to 218 µm or between 219 µm to 234 µm or between 237 µm to 253 µm or between 257 µm to 268 µm or between 269 µm to 278 µm or between 283 µm to 298 µm.

According to one embodiment of this invention, brivaracetam has a d (0.1) particle size between 1 µm to 40 µm or between 4 µm to 34 µm or between 6 µm to 25 µm.

According to one embodiment of this invention, brivaracetam has a d (0.5) particle size between 8 µm to 85 µm or between 13 µm to 76 µm or between 22 µm to 65 µm or between 27 µm to 58 µm.

According to one embodiment of this invention, brivaracetam has a d (0.1) particle size between 1 µm to 40 µm and a d (0.5) particle size between 8 µm to 85 µm and a d (0.9) particle size between 100 µm to 300 µm.

According to an embodiment of the present invention, at least one pharmaceutically acceptable excipient is selected from the group comprising pH adjusters, antimicrobial agents, sweetening agents, tonicity adjusting agents, flavoring agents or mixtures thereof.

According to an embodiment of the present invention, the pH adjuster has sufficient capacity to stay within the target pH range when diluted with neutral, weakly acidic or weakly basic liquid.

Suitable pH adjusters are selected from the group comprising malic acid, sodium citrate, anhydrous citric acid, tartaric acid, sodium potassium tartrate, aluminum potassium sulfate, anhydrous disodium hydrogen phosphate, potassium carbonate, anhydrous sodium dihydrogen phosphate, dibasic potassium sulfate, calcium carbonate, nicotinic acid, dilute hydrochloric acid, glacial acetic acid, lactic acid, maleic acid, monobasic potassium phosphate, phosphoric acid, adipic acid, sodium acetate, sodium bicarbonate, sodium carbonate, sodium dihydrogen phosphate dihydrate, tribasic sodium phosphate or mixtures thereof.

According to one embodiment of this invention, the pH adjuster agent is sodium citrate, anhydrous citric acid or mixtures thereof. Further studies have found that the pH adjuster agents provide good color stability of the solution. The pH adjusters have sufficient capacity to stay within the target pH range when diluted with neutral, weakly acidic or weakly basic liquid. The target pH is 5.5 ± 2.

Suitable antimicrobial agents are selected from the group comprising methyl paraben, propyl paraben, benzyl alcohol, benzoic acid, boric acid, sorbic acid and their salts thereof, benzalkonium chloride or mixtures thereof.

According to one embodiment of this invention, the antimicrobial agent is methyl paraben.

Suitable sweetening agents are selected from the group comprising sucralose, sorbitol solution 70%, sodium cyclamate, acesulfame potassium, dipotassium glycyrrhizate, stevia extracts, saccharin sodium, sucrose, xylitol, mannitol, erythritol or mixtures thereof.

According to one embodiment of this invention, the sweetening agent is sucralose, sorbitol solution 70% or mixtures thereof.

Suitable tonicity adjusting agents is selected from the group comprising glycerin, sodium chloride, potassium chloride, calcium chloride, mannitol, glycerol, sorbitol, propylene glycol, dextrose, sucrose or mixtures thereof.

According to one embodiment of this invention, the tonicity adjusting agent is glycerin.

Suitable flavoring agents are selected from the group comprising menthol, peppermint, cinnamon, chocolate, vanillin or fruit essences such as cherry, orange, strawberry, grape, black currant, raspberry, banana, red fruits, wild berries or mixtures thereof.

According to one embodiment of this invention, the flavoring agent is raspberry, strawberry or mixtures thereof.

According to one embodiment of this invention, the oral solution comprises;
- Brivaracetam
- At least one cellulose derivative excipient
- Sodium citrate
- Anhydrous citric acid
- Methylparaben

According to one embodiment of this invention, the oral solution comprises;
- Brivaracetam
- At least one cellulose derivative excipient
- Sodium citrate
- Anhydrous citric acid
- Methylparaben
- Sucralose
- Sorbitol solution 70%
- Glycerin
- Raspberry flavor

Suitable solvents are selected from the group comprising pure water, propylene glycol, ethanol, polyethylene glycol or mixtures thereof.

In the present invention, a process operation is simple, and it is easy to prepare, good repeatability of preparation sample, for producing cost is not high.

A process for the preparation of the oral solution comprising brivaracetam comprises the following steps:
a) Sodium citrate, anhydrous citric acid, methyl paraben, propyl paraben, sucralose and brivaracetam is dissolved in water,
b) At least one cellulose derivatives excipient is added and dissolved in solution obtained in step-a.
c) Sorbitol solution added and mixed,
d) Glycerin added and mixed,
e) Raspberry flavor is added and mixed,
f) solution is marked up to the volume with purified water.

### Example 1: Oral solution

| **Ingredients** | **% by weight** |
|---|---|
| Brivaracetam | 0.89 |
| Sodium citrate | 0.26 |
| Anhydrous citric acid | 0.08 |
| Methylparaben | 0.09 |
| Avicel RC / CL (MCC & Sodium carboxymethyl cellulose) | 0.45 |
| Sucralose | 3.58 |
| Sorbitol solution 70% | 30.70 |
| Glycerin | 13.60 |
| Flavor | 0.50 |
| Solvent | 49.85 |
| **Total** | **100** |

A process for example 1;
a) Sodium citrate, anhydrous citric acid, methyl paraben, propyl paraben, sucralose and brivaracetam is dissolved in water,
b) Avicel RC / CL is added and dissolved in solution obtained in step-a.
c) Sorbitol solution added and mixed,
d) Glycerin added and mixed,
e) Raspberry flavor is added and mixed,
f) solution is marked up to the volume with purified water.

### Example 2: Oral solution

| **Ingredients** | **% by weight** |
|---|---|
| Brivaracetam | 0.89 |
| Sodium citrate | 0.26 |
| Anhydrous citric acid | 0.08 |
| Methylparaben | 0.09 |
| HPMC E 5 LV | 0.45 |
| Sucrolose | 3.58 |
| Sorbitol solution 70% | 30.70 |
| Glycerin | 13.60 |
| Flavor | 0.50 |
| Solvent | 49.85 |
| **Total** | **100** |

A process for example 2;
a) Sodium citrate, anhydrous citric acid, methyl paraben, sucralose and brivaracetam is dissolved in water,
b) HPMC E5LV is added and dissolved in solution obtained in step-a.
c) Sorbitol solution added and mixed,
d) Glycerin added and mixed,
e) Strawberry flavor is added and mixed,
f) solution is marked up to the volume with purified water.

### Example 3: Oral solution

| **Ingredients** | **% by weight** |
|---|---|
| Brivaracetam | 0.89 |
| Sodium citrate | 0.26 |
| Anhydrous citric acid | 0.08 |
| Methylparaben | 0.09 |
| HPC | 0.45 |
| Sucralose | 3.58 |
| Sorbitol solution 70% | 30.70 |
| Glycerin | 13.60 |
| Flavor | 0.50 |
| Solvent | 49.85 |
| **Total** | **100** |

A process for example 3;
a) Sodium citrate, anhydrous citric acid, methyl paraben, sucralose and brivaracetam is dissolved in water,
b) HPC is added and dissolved in solution obtained in step-a.
c) Sorbitol solution added and mixed,
d) Glycerin added and mixed,
e) Raspberry flavor is added and mixed,
f) Solution is marked up to the volume with purified water.

## Claims

1. An oral solution comprising brivaracetam and at least one pharmaceutically acceptable excipient wherein at least one cellulose derivative excipient is present.

2. The oral solution according to claim 1, wherein is cellulose derivative excipient is a viscosity agent which is microcrystalline cellulose, sodium carboxymethyl cellulose, hydroxypropyl methyl cellulose, hydroxypropyl cellulose or mixtures thereof.

3. The oral solution according to claim 2, wherein the viscosity agents are microcrystalline cellulose and sodium carboxymethyl cellulose.

4. The oral solution according to claim 2, wherein the viscosity agent is hydroxypropyl methyl cellulose.

5. The oral solution according to claim 2, wherein the viscosity agent is hydroxypropyl cellulose.

6. The oral solution according to claim 1, wherein the amount of brivaracetam is 0.1% to 7.0% by weight in the total composition.

7. The oral solution according to claim 1, wherein the amount of brivaracetam is 0.1% to 5.0% by weight in the total composition, preferably 0.2% to 3.0% by weight in the total composition, preferably 0.3% to 1.8% by weight in the total composition.

8. The oral solution according to claim 1, wherein brivaracetam has a d (0.9) particle size between 100 µm to 300 µm.

9. The oral solution according to claim 1, wherein brivaracetam has a d (0.1) particle size between 1 µm to 40 µm or between 4 µm to 34 µm or between 6 µm to 25 µm.

10. The oral solution according to claim 1, wherein brivaracetam has a d (0.5) particle size between 8 µm to 85 µm or between 13 µm to 76 µm or between 22 µm to 65 µm or between 27 µm to 58 µm.

11. The oral solution according to claim 1, wherein brivaracetam has a d (0.1) particle size between 1 µm to 40 µm and a d (0.5) particle size between 8 µm to 85 µm and a d (0.9) particle size between 100 µm to 300 µm.

12. The oral solution according to claim 1, wherein at least one pharmaceutically acceptable excipient is selected from the group comprising pH adjusters, antimicrobial agents, sweetening agents, tonicity adjusting agents, flavoring agents or mixtures thereof.

13. The oral solution according to claim 1, wherein the oral solution comprises;
• Brivaracetam
• At least one cellulose derivative excipient
• Sodium citrate
• Anhydrous citric acid
• Methylparaben

14. The oral solution according to claim 1, wherein the oral solution comprising;
• Brivaracetam
• At least one cellulose derivative excipient
• Sodium citrate
• Anhydrous citric acid
• Methylparaben
• Sucralose
• Sorbitol solution 70%
• Glycerin
• Raspberry flavor
